(19) Europäisches Patentamt
European Patent Office
Office européen des brevets

(11)  EP 1 584 618 B1

(12)  **EUROPEAN PATENT SPECIFICATION**

(45) Date of publication and mention
of the grant of the patent:
**22.08.2007   Bulletin 2007/34**

(51) Int Cl.:
***C07D 213/79*** *(2006.01)*     ***C07D 213/807*** *(2006.01)*
***B01J 23/22*** *(2006.01)*

(21) Application number: **04015397.5**

(22) Date of filing: **30.06.2004**

(54) **Process for preparing nicotinic acid and catalyst used in the method**

Verfahren zur Herstellung von Nicotinsäure und in dem Verfahren verwendeter Katalysator

Procédé de préparation d'acide nicotinique et catalyseur utilisé dans le procédé

(84) Designated Contracting States:
**AT BE BG CH CY CZ DE DK EE ES FI FR GB GR HU IE IT LI LU MC NL PL PT RO SE SI SK TR**

(30) Priority: **08.04.2004   CN 04130796**

(43) Date of publication of application:
**12.10.2005   Bulletin 2005/41**

(73) Proprietor: **Chang Chun Petrochemical Co. Ltd. Tapei, Taiwan (TW)**

(72) Inventors:
  • **Lu, Chih-Sheng**
    **Miaoli,**
    **Taiwan (TW)**
  • **Lee, Chao-Yang**
    **Miaoli,**
    **Taiwan (TW)**
  • **Hsiung, Shih-Chin**
    **Miaoli,**
    **Taiwan (TW)**
  • **Tsai, Jing-Jin**
    **Miaoli,**
    **Taiwan (TW)**

(74) Representative: **Behnisch, Werner et al**
    **Reinhard-Skuhra-Weise & Partner GbR**
    **Friedrichstrasse 31**
    **80801 München (DE)**

(56) References cited:
    **DE-A1- 2 647 712          DE-A1- 10 146 088**
    **DE-A1- 19 822 788**

  • **HEINZ D ET AL: "V2O5/TiO2 Catalysts for the Vapor-Phase Oxidation of beta-Picoline: Influence of the TiO2-Carrier" JOURNAL OF CATALYSIS, ACADEMIC PRESS, DULUTH, MN, US, vol. 192, no. 1, 15 May 2000 (2000-05-15), pages 1-10, XP004437979 ISSN: 0021-9517**

**Description**

Field of the invention

The present invention relates to a process for preparing nicotinic acid, which comprises directly subjecting a mixture of 3-methylpyridine, oxygen, and water to a vapor phase oxidation in the presence of a catalyst consisting of, as active ingredients, vanadium oxide ($V_2O_5$) and transition metal oxide both of which are supported on a carrier, to give the nicotinic acid, wherein the crystal size of the active ingredients on the surface of the carrier is controlled in a range of from 40 to 200 nm through use of the transition metal oxide.

The present invention also relates to a process for preparing nicotinic acid, which further comprises the steps of scrubbing the resulting nicotinic acid and un-reacted 3-methylpyridine into water and then distilling the mixture to distill off the un-reacted 3-methylpyridine and recycle to next process.

The present invention further relates to a catalyst used in the vapor phase oxidation.

Prior art of the invention

Nicotinic acid and its derivatives have been widely used as vitamins, drugs, and plant grow regulator in pharmaceutical and agricultural fields due to their various physiological properties.

The current available process for producing nicotinic acid mainly includes liquid phase reaction and vapor phase reaction. Among the liquid phase reaction, the following methods have been disclosed:

1. GB568889 discloses a process for preparing nicotinic acid by liquid phase oxidation, which comprises subjecting 3-methylpyridine to oxidation by adding concentrated sulfuric acid and concentrated nitric acid as oxidants, then neutralizing and purifying the resulting nicotinic acid. However, this process will produce a lot of $NO_x$ and should add a lot of base to neutralize the added inorganic acid during the purification step. Thus, it produces a lot of waste material. Moreover, in the neutralization procedure the inorganic salt forms, which is difficult to be removed from the reaction thoroughly. Also, this process encountered a problem of equipment corrosion due to the use of strong acids as oxidants.

2. USP 4,217,457 disclosed a process for producing pyridine carboxylic acid (also refer to "nicotinic acid") in high selectivity, which comprises subjecting alkyl pyridine to oxidation by using hexa valence chromate as an oxidant to prepare pyridine carboxylic acid. This process encountered a problem of producing a lot of tri valence chromium during oxidation. The resulting tri valence chromium is not environmentally benign. Also, in purification an inorganic acid or an organic acid should be added which in turn produce a lot of salts.

3. G8824293 and USP 5,700,944 each disclosed a process for producing pyridine carboxylic acid (also refer to "nicotinic acid"), which comprises subjecting alkyl pyridine to liquid phase oxidation in acetic acid in the presence of cobalt acetate, manganese acetate, and bromine-containing tertiary ammonium salt as catalysts at an elevated temperature under high pressure to give pyridine carboxylic acid. These processes encountered a problem of producing bromine due to addition of bromine-containing tertiary ammonium salt. Thus, a further purification for removing bromine by using catalyst is necessary. It complicates the process and increases manufacturing cost.

The common disadvantages of the above liquid phase oxidation process for producing nicotinic acid is producing lots kinds of by-products which are not environmentally benign.

As to the vapor phase oxidation, there are two kinds of reactions. One is ammoxidation process and the other is direct oxidation process. So far the ammoxidation process is popular. Such processes are now described as follows.

1. USP 3,803,156 disclosed a process for producing pyridine carboxylic acid, which comprises contacting methylpyridine, molecular oxygen-containing gas, and water with solid oxidation catalyst containing a vanadium compound bonded with oxygen in the vapor phase to produce pyridine carboxylic acid. In this process, minor amount of oxides of germanium, tin, indium, niobium, tantalum, gallium, and zirconium are used as promoter. This process has disadvantages of high reaction temperature and a large amount of water which in turn increases the energy consumption during purification.

2. USP 5,719,045 disclosed a process for producing nicotinamide, which comprises firstly catalytically converting 2-methyl-1,5-diaminopentane into 3-picoline by using aluminum oxide and silicon oxide as catalysts, then subjecting the resultant 3-picoline to ammoxidation by using oxides of vanadium, titanium, zirconium and molybdenum as ammoxidation catalysts to convert into 3-cyanopyridine, and finally microbiologically converting the 3-cyanopyridine into the nicotinamide through the use of *Rhodococcus.*

3. USP 5,728,837 disclosed a process for producing nicotinic acid, which comprises subjecting 3-methylpyridine and water to vapor oxidation in the presence of vanadium- and titanium-based oxides as catalyst with or without additional additives to produce nicotinic acid.

4. USP 6,229,018 disclosed a process for producing nicotinic acid, which comprises catalytically oxidizing 3-methylpyridine with oxygen-containing gas and water to nicotinic acid. In this process, water and 3-methylpyridine are separately fed into catalyst bed. The catalyst is a vanadium oxide ($V_2O_5$) in amount of 5 to 50 % by weight supported on titanium oxide. The catalyst is produced by sulfuric acid method and the supporter titanium oxide has high specific surface area. This patent mentioned that if the oxidation uses the carrier titanium oxide having a specific surface area more than 250 $m^2$/g, the amount of the vanadium oxide obtained should be in an amount of at least 20 % by weight, based on the weight of the catalyst. Otherwise a desired yield could not be achieved. If the oxidation uses titanium oxide having low specific surface area and vanadium oxide in low amount, the yield of the nicotinic acid will reduce. As shown in Example 1 in USP 6,229,018, by using titanium oxide P-25 having a specific surface area of 50 $m^2$/g and being free of sulfate salt and using vanadium oxide in amount of 2.5%, the conversion of methylpyridine is only 61.6% and selectivity is only 22%.

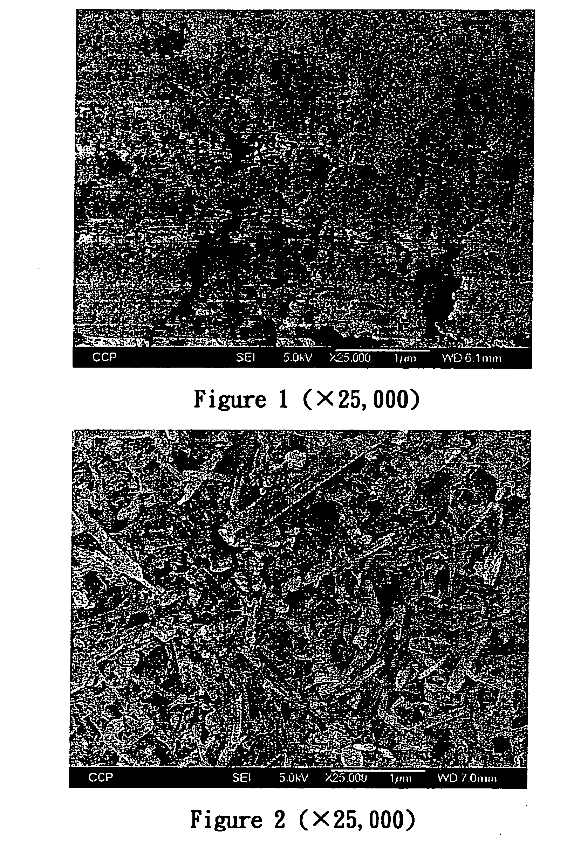

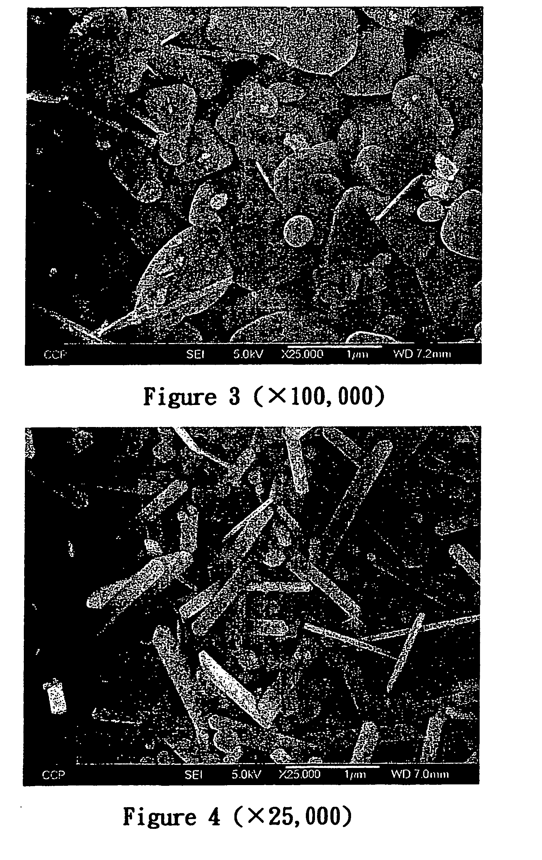

In view of the poor selectivity and conversion or a large amount of vanadium oxide required in the above prior vapor phase process for producing nicotinic acid, the present inventors have conducted an investigation on the process for producing nicotinic acid and found that such processes all use catalyst containing vanadium oxide as active ingredient and titanium oxide as a carrier. However, a crystal size of the vanadium oxide varies with different temperature. As demonstrated in Comparative Example 1 mentioned hereafter, after conducting vapor phase oxidation of methylpyridine by using a catalyst consisting of vanadium oxide as active ingredient and titanium oxide as a carrier, it was found that the conversion of methylpyridine decreased while time passed. On 13$^{th}$ day of the reaction, the catalyst was analyzed by scanning electronic microscope and was found that the crystal size of the vanadium oxide on the surface of the carrier grew up. As shown in Figures 1 to 3, Figure 1 shows a photograph of the catalyst before oxidation reaction, in which the crystal size is approximately 10 to 20 nm. Figure 2 shows a photograph of the catalyst after oxidation reaction for 13 days, in which the crystal size grew up to 200 to 300 nm. Figure 3 shows a photograph of some of the catalyst after oxidation reaction for 13 days, in which the crystal size even grew up to more than 1 $\mu$m. The present inventors draw an inference that the reason for decreasing of the conversion may be due to the increased crystal size which resulting in lowered specific surface area and inferior activity.

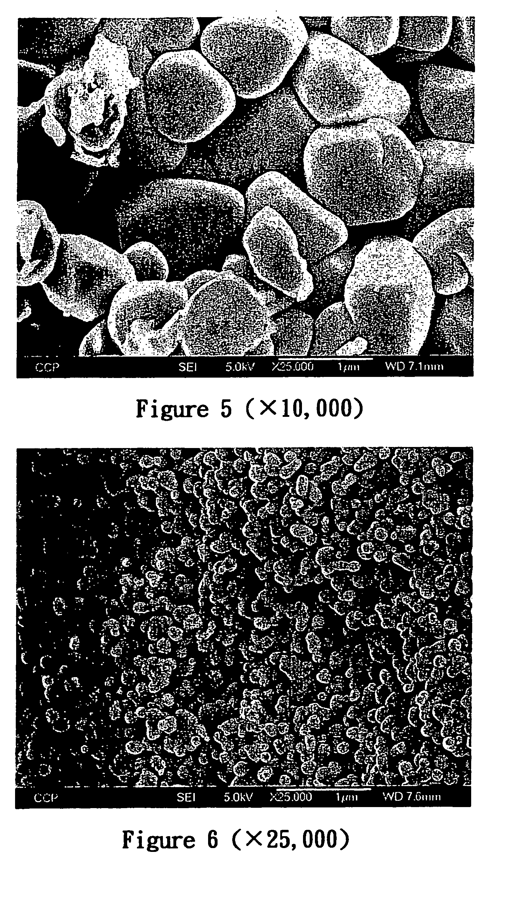

Accordingly, the present inventors conducted an experiment for comparing an activity of a catalyst containing 20% vanadium oxide supported on titanium oxide at different temperature. As a result, it is found that the crystal size of vanadium oxide will grow as needle crystal when calcined at a temperature above 450°C, as shown in Figure 4. Further, the crystal size of vanadium oxide will grow as round crystal when calcined at a temperature above 690°C, as shown in Figure 5. Such a crystal size variation is considered as a possible reason resulting in inferior yield and poor selectivity.

In JOURNAL OF CATALYSIS, Vol. 192, No.1, pages 1-10, a process is described for preparing nicotinic acid starting from 3-methylpyridine, air and water in the presence of a catalyst consisting of vanadium oxide on $TiO_2$ as support. However, this document mainly studied the effect of the type of $TiO_2$ carrier on the activity of the V/Ti-oxide and found that anatase type of $TiO_2$-carrier has a positive effect on the vanadia layer. According to its disclosure, adding a metal oxide such as $CeO_2$ does not lead to a better catalytic performance in this reaction.

Summary of the Invention

The first objective of the present invention is to provide a process for preparing nicotinic acid, which comprises subjecting a mixture of 3-methylpyridine, oxygen, and water to a vapor phase oxidation at a temperature of from 250°C to 350°C in the presence of a catalyst consisting of, as active ingredients, vanadium oxide ($V_2O_5$) and transition metal oxide, both of which are supported on a support, to give the nicotinic acid, wherein the catalyst is produced from calcining and drying of ammonium meta-vanadate and transition metallate salts supported on a carrier, and the crystal size of the active ingredients on the surface of the carrier is controlled in a range of from 40 to 200 nm through use of the transition metal oxide.

The second objective of the present invention is to provide a process for preparing nicotinic acid, which comprises subjecting a mixture of 3-methylpyridine, oxygen, and water to a vapor phase oxidation at a temperature of from 250°C to 350°C in the presence of a catalyst consisting of vanadium oxide ($V_2O_5$) and transition metal oxide, both of which are supported on a support, to give the nicotinic acid, then scrubbing the resulting nicotinic acid and un-reacted 3-methylpyridine into water, and distilling the resulting aqueous solution at an overhead temperature of from 96°C to 100°C to distill off the 3-methylpyridine and recycle to next process, wherein the catalyst is produced from calcining and drying of ammonium meta-vanadate and transition metallate salts supported on a carrier, and the crystal size of the active ingredients on the surface of the carrier is controlled in a range of from 40 to 200 nm through use of transition metal oxide.

In the process of the first and second objective of the present invention, a mole ratio of 3-methylpyridine to oxygen is from 1:15 to 1:60, a mole ratio of 3-methylpyridine to water is from 1:70 to 1:350, and the 3-methylpyridine is fed into the reaction at a WHSV (Weight Hourly Space Velocity) of from 0.01 to 0.1 hr$^{-1}$. The WHSV is defined as follows.

$$\text{WHSV=Feed rate of 3-methylpyridine (kg/hr)/Catalyst weight(kg)}$$

The third objective of the present invention is to provide a catalyst used in the above oxidation process, which consists of vanadium oxide ($V_2O_5$) and transition metal oxide both of which are supported on a carrier, in which a crystal size of the active ingredients on the surface of the carrier is in a range of from 40 to 200 nm.

In the catalyst used in the above oxidation process, the crystal size of the active ingredients on the surface of the carrier is preferably in a range of from 40 to 100 nm.

In the present invention, the term "active ingredients" used herein is intended to mean vanadium oxide ($V_2O_5$), transition metal oxide, or the both.

In the process of the present invention, the term "oxygen" means any gas containing oxygen, such as air or pure oxygen.

In the catalyst of the present invention, vanadium oxide functions as a main catalyst and the transition metal oxide functions as a co-catalyst in addition to the function for controlling the crystal size of the active ingredients on the surface of the carrier.

In the catalyst of the present invention, the transition metal oxide is one or more metal oxides selected from the group consisting of chromium oxide, molybdenum oxide, tungsten oxide, manganese oxide, ferric oxide, cobalt oxide, nickel oxide, cupric oxide, and zinc oxide.

According to the catalyst of the present invention, by adding the transition metal oxide to control the crystal size of the active ingredients on the surface of the carrier in a range of from 40 to 200 nm, preferably from 40 to 100 nm, the vanadium oxide contained in the catalyst could be used in less amount to carry out the process for producing nicotinic acid in the present invention. Also, a stability of the catalyst is increased and its life prolongs, which will in turn lower the cost for producing nicotinic acid.

Additionally, according to the process of the first and second objectives of the present invention, by using the catalyst of the present invention, a conversion of the 3-methylpyridine will increase to at least 88% and the selectivity of the nicotinic acid will increase to at least 88%.

The catalyst of the present invention is prepared by the following process. First, dissolve ammonium meta-vanadate in a solvent, and then add a transition metallate salt and stir thoroughly, then add a carrier to adsorb the solution containing ammonium meta-vanadate and transition metallate salt. Heat the resulting carrier and evaporate the solvent, then calcine the carrier at a temperature of from 450°C to 800°C, preferably at a temperature of from 450°C to 700°C to calcine the ammonium meta-vanadate and transition metallate salt, to obtain a catalyst consisting of vanadium oxide and transition metal oxide supported on the carrier.

In the process of the present invention, the transition metallate salts are inorganic salts of one or more transition metal selected from the group consisting of chromium, molybdenum, tungsten, manganese, ferric, cobalt, nickel, copper, and zinc. Examples of the transition metallate salts include, for example, ammonium chromate, sodium chromate, potassium chromate, calcium chromate, magnesium chromate, chromium nitrate, chromium sulfate, chromium hydroxide, ammonium molybdenate, sodium molybdenate, potassium molybdenate, calcium molybdenate,, magnesium molybdenate, ammonium tungstate, sodium tungstate, potassium tungstate, calcium tungstate, magnesium tungstate, ammonium permanganate, sodium permanganate, potassium permanganate, calcium permanganate, magnesium permanganate, ferric nitrate, ferric sulfate, zinc nitrate, and zinc sulfate.

In the catalyst according to the present invention, the carrier can use the carrier commonly used in catalyst field. Examples of the carrier include titanium oxide, silica oxide, and aluminum oxide, with titanium oxide is preferable.

In the catalyst according to the present invention, the amounts of ammonium meta-vanadate, transition metallate salt, and the carrier are controlled such that after calcination the amount of vanadium oxide is from 2.5 to 20% by weight and the amount of transition metal oxide is from 0.1 to 10% by weight, based on the total weight of the vanadium oxide, transition metal oxide, and the carrier.

Brief Description of the Drawings

Figure 1 is an electronic microscopic photograph showing the catalyst used in Comparative Example 1 before oxidation reaction;

Figure 2 is an electronic microscopic photograph showing the catalyst used in Comparative Example 1 after oxidation reaction for 13 days;

Figure 3 is an electronic microscopic photograph showing the catalyst used in Comparative Example 1 after oxidation reaction for 13 days;

Figure 4 is an electronic microscopic photograph showing the catalyst consisting of 20% by weight of vanadium oxide and titanium oxide carrier after calcination at 500°C;

Figure 5 is an electronic microscopic photograph showing the catalyst consisting of 20% by weight of vanadium oxide and titanium oxide carrier after calcination at 700°C ;

Figure 6 is an electronic microscopic photograph showing the catalyst used in Example 1 after calcinations, before oxidation reaction;

Figure 7 is an electronic microscopic photograph showing the catalyst used in Example 1 after oxidation reaction for 42 days;

Figure 8 is an electronic microscopic photograph showing the catalyst used in Example 2 after calcinations, before oxidation reaction;

Figure 9 is an electronic microscopic photograph showing the catalyst used in Example 3 after calcinations, before oxidation reaction;

Figure 10 is an electronic microscopic photograph showing the catalyst used in Example 4 after calcinations,before oxidation reaction; and

Figure 11 is an electronic microscopic photograph showing the catalyst used in Example 5 after calcinations,before oxidation reaction.

Detailed Description of the Invention

The present invention is now described in more detail by reference to the following Examples. The Examples are only used for illustrating the present invention without limiting the scope of the present invention.

Comparative Example 1

Process for preparing nicotinic acid by using conventional catalyst

30 Grams of vanadium oxide and 12 grams titanium oxide carrier (KataLeuna KL4500-TL 1.6) were added into a tube reactor having a diameter of 1 inch and a length of 5 centimeter to obtain a catalyst bed. 3-Methylpyridine was mixed with air then water vapor to give a mixture. The resultant mixture was continuously fed into the catalyst bed heated at a temperature of 260°C. The mole ratio of 3-methylpyridine, oxygen contained in the air, and water was 3-methylpyridine: oxygen: water of 1:30:70, and the feed velocity of 3-methylpyridine was 0.025 $hr^{-1}$. On day 1, a conversion of 3-methylpyridine was 88.96%, a selectivity of nicotinic acid was 75.65%. After continuous reacting for 13 days, a conversion of 3-methylpyridine reduced to 68.2% and a selectivity of nicotinic acid was 78.74%.

In this reaction, the amount of nicotinic acid was determined by High Performance Liquid Chromatography (HPLC) by using C-18 column as a separation column. The nicotinic acid was first sampled from reactor and rinsed with water, and then injected into HPLC and quantified.

Also, un-reacted 3-methylpyridine and carbon dioxide formed during reaction were quantified by Gas Chromatography (GC).

After continuous reacting for 13 days, the catalyst was sampled and analyzed by electronic microscopy. Its electronic microscopic photograph is shown in Figures 2 and 3. The photograph of the catalyst before reaction is shown in Figure 1. By comparing Figures 1 to 3, it is known that the crystal size of the active ingredient (vanadium oxide) on the surface of the carrier gradually grow up while time passed. The increased crystal size is a possible reason of lowering conversion.

Example 1 The preparation of the catalyst of the present invention and the process for preparing nicotinic acid by using the catalyst

6.43 g of ammonium meta-vanadate were added into 500 ml water and the solution was heated at 70°C to dissolve ammonium meta-vanadate. Then, 5.46 g of ammonium chromate were added into the solution and stirred for 30 minutes. Into the resultant solution were added 91.41 g titanium oxide (Hembitec K-03) and stirred for 1 hour. The mixture was heated to evaporate water and then calcined in an oven at a temperature of 700°C to obtain the catalyst of the present invention, whose composition was shown in Table 1. After calcination, the catalyst was observed by scanning electronic microscopy and was found that the crystal size of the active ingredients on the surface of the carrier is from 80 to 100 nm, as shown in Figure 6.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:45:145 (3-methylpyridine: oxygen: $H_2O$) and where the bed temperature was controlled at 290°C. The feed speed of 3-methylpyridine is 0.025 $hr^{-1}$. The product was collected from output of the catalyst bed and analyzed by HPLC and GC. It was found that a conversion of 3-methylpyridine is 96.82%, a selectivity of nicotinic acid is 93.16%, and a selectivity of carbon dioxide is 6.76%.

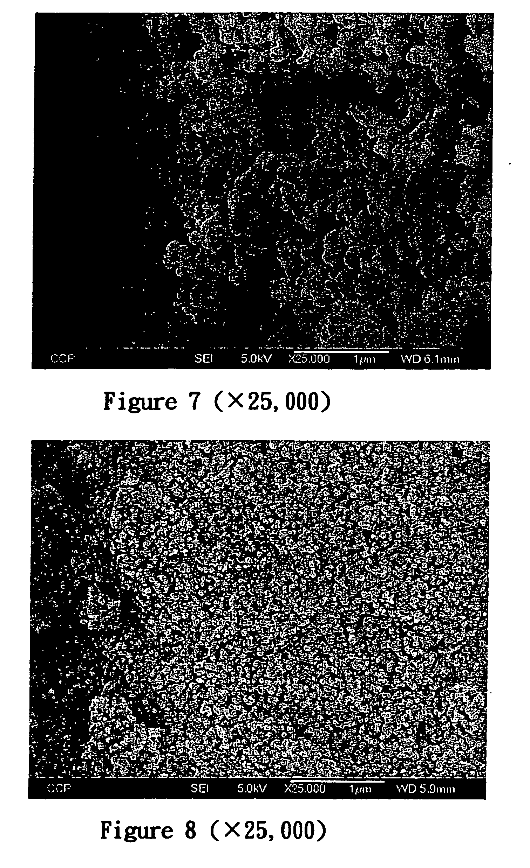

After continuous processing for 42 days, the catalyst was drawn out and examined by electronic microscopy. Its microscopic photograph was shown in Figure 7. From the Figure, it is known that according to the present process for preparing nicotinic acid by using the present catalyst, the crystal size of the active ingredients on the surface of carrier did not vary while time passed. Thus it demonstrates that the catalyst of the present invention exhibits excellent stability and longer lifetime. Moreover, as the crystal size of the active ingredients on the surface of the carrier is controlled in the range of from 40 to 100 nm by adding transition metal oxide, its catalytic activity increases. Thus a desired conversion and selectivity will be achieved by using less amount of catalyst.

Example 2 The preparation of the catalyst of the present invention and the process for preparing nicotinic acid by using the catalyst

6.43 g of ammonium meta-vanadate were added into 500 ml water and the solution was heated at 70°C to dissolve ammonium meta-vanadate. Then, 3.44 g of ammonium molybdenate were added into the solution and stirred for 30 minutes. Into the resultant solution were added 92.22 g titanium oxide (Hembitec K-03) and stirred for 1 hour. The mixture was heated to evaporate water and then calcined in an oven at a temperature of 600°C to obtain the catalyst of the present invention, whose composition was shown in Table 1. After calcination, the catalyst was observed by electronic microscopy and found that the crystal size of the active ingredients on the surface of the carrier is from 40 to 60 nm, as shown in Figure 8.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:40:170 (3-methylpyridine: oxygen: $H_2O$) and where the bed temperature was controlled at 300°C. The feed speed of 3-methylpyridine is 0.021 hr$^{-1}$. The product was collected at the outlet of the catalyst bed and analyzed by HPLC and GC. It was found that a conversion of 3-methylpyridine is 90.21 %, a selectivity of nicotinic acid is 90.18%, and a selectivity of carbon dioxide is 8.54%.

Example 3 The preparation of the catalyst of the present invention and the process for preparing nicotinic acid by using the catalyst

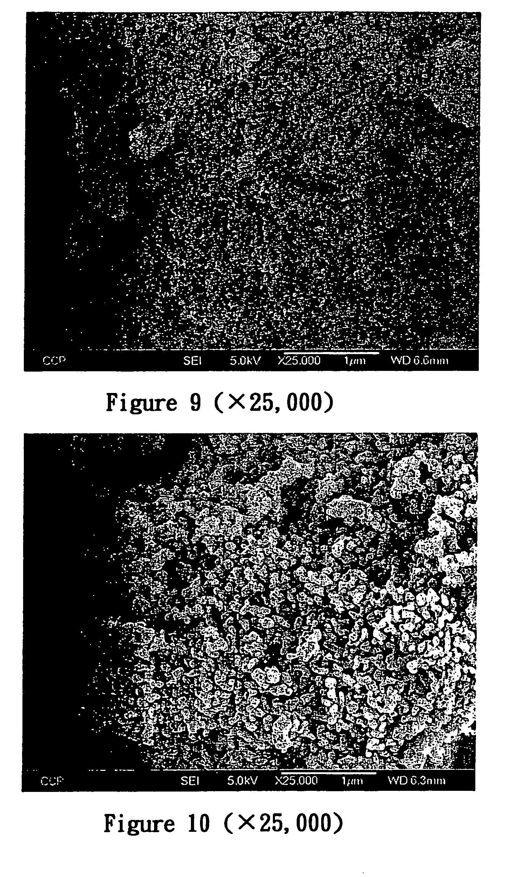

6.43 g of ammonium meta-vanadate were added into 500 ml water and the solution was heated at 70°C to dissolve ammonium meta-vanadate. Then, 2.5 g of ammonium tungstate were added into the solution and stirred for 30 minutes. Into the resultant solution were added 92.65 g titanium oxide (Hembitec K-03) and stirred for 1 hour. The mixture was heated to evaporate water and then calcined in an oven at a temperature of 600°C to obtain the catalyst of the present invention, whose composition was shown in Table 1. After calcination, the catalyst was observed by electronic microscopy and found that the crystal size of the active ingredients on the surface of the carrier is from 40 to 60 nm, as shown in Figure 9.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:37:160 (3-methylpyridine: oxygen: $H_2O$) and where the bed temperature was controlled at 285°C . The feed speed of 3-methylpyridine is 0.028 hr$^{-1}$. The product was collected at the outlet of the catalyst bed and analyzed by HPLC and GC. It was found that a conversion of 3-methylpyridine is 92.83%, a selectivity of nicotinic acid is 92.22%, and a selectivity of carbon dioxide is 7.11%.

Example 4 The preparation of the catalyst of the present invention and the process for preparing nicotinic acid by using the catalyst

6.43 g of ammonium meta-vanadate were added into 500 ml water and the solution was heated at 70°C to dissolve ammonium meta-vanadate. Then, 5.37 g of ammonium permanganate were added into the solution and stirred for 30 minutes. Into the resultant solution were added 91.5 g titanium oxide (Hembitec K-03) and stirred for 1 hour. The mixture was heated to evaporate water and then calcined in an oven at a temperature of 600°C to obtain the catalyst of the present invention, whose composition was shown in Table 1. After calcination, the catalyst was observed by scanning electronic microscopy and found that the crystal size of the active ingredients on the surface of the carrier is from 40 to 60 nm, as shown in Figure 10.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:20:150 (3-methylpyridine: oxygen: $H_2O$) and where the bed temperature was controlled at 305°C. The feed speed of 3-methylpyridine is 0.025 hr$^{-1}$. The product was collected at the outlet of the catalyst bed and analyzed by HPLC and GC. It was found that a conversion of 3-methylpyridine is 95.67%, a selectivity of nicotinic acid is 89.84%, and a selectivity of carbon dioxide is 8.98%.

Example 5 The preparation of the catalyst of the present invention and the process for preparing nicotinic acid by using the catalyst

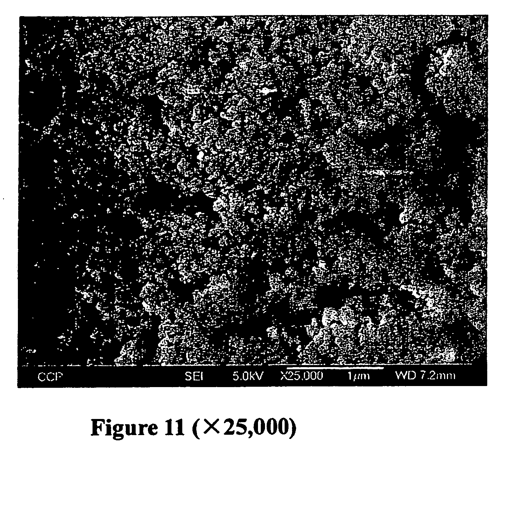

6.43 g of ammonium meta-vanadate were added into 500 ml water and the solution was heated at 70°C to dissolve ammonium meta-vanadate. Then, 13.54 g of ferric nitrate were added into the solution and stirred for 30 minutes. Into the resultant solution were added 92.31 g titanium oxide (Hembitec K-03) and stirred for 1 hour. The mixture was heated to evaporate water and then calcined in an oven at a temperature of 700°C to obtain the catalyst of the present invention, whose composition was shown in Table 1. After calcination, the catalyst was observed by electronic microscopy and found that the crystal size of the active ingredients on the surface of the carrier is from 60 to 80 nm, as shown in Figure 11.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:35:330 (3-methylpyridine: oxygen: $H_2O$) and where the bed temperature was controlled at 310°C. The feed speed of 3-methylpyridine is 0.02 $hr^{-1}$. The product was collected at the outlet of the catalyst bed and analyzed by HPLC and GC. It was found that a conversion of 3-methylpyridine is 96.78%, a selectivity of nicotinic acid is 93.13%, and a selectivity of carbon dioxide is 5.56%.

Example 6 The preparation of the catalyst of the present invention and the process for preparing nicotinic acid by using the catalyst

6.43 g of ammonium meta-vanadate were added into 500 ml water and the solution was heated at 70°C to dissolve ammonium meta-vanadate. Into the resultant solution were added 92.27 g titanium oxide (Hembitec K-03) and stirred for 30 minutes to obtain slurry containing ammonium meta-vanadate and titanium oxide. Separately, 14.37g of chromium nitrate was dissolved in water and slowly added into the slurry. The mixture was stirred for 1 hour, heated to evaporate water, and then calcined in an oven at a temperature of 600°C to obtain the catalyst of the present invention, whose composition was shown in Table 1. After calcination, the catalyst was observed by electronic microscopy and found that the crystal size of the active ingredients on the surface of the carrier is from 60 to 80 nm.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:40:170 (3-methylpyridine: oxygen: $H_2O$) and where the bed temperature was controlled at 300°C. The feed speed of 3-methylpyridine is 0.021 $hr^{-1}$. The product was collected at the outlet of the catalyst bed and analyzed by HPLC and GC. It was found that a conversion of 3-methylpyridine is 93.54%, a selectivity of nicotinic acid is 93.16%, and a selectivity of carbon dioxide is 6.39%.

Example 7 The preparation of the catalyst of the present invention and the process for preparing nicotinic acid by using the catalyst

6.43 g of ammonium meta-vanadate were added into 500 ml water and the solution was heated at 70°C to dissolve ammonium meta-vanadate. Then, 8.2 g of zinc sulfate were added into the solution and stirred for 30 minutes. Into the resultant solution were added 92.68 g titanium oxide (Hembitec K-03) and stirred for 1 hour. The mixture was heated to evaporate water and then calcined in an oven at a temperature of 600°C to obtain the catalyst of the present invention, whose composition was shown in Table 1. After calcination, the catalyst was observed by electronic microscopy and found that the crystal size of the active ingredients on the surface of the carrier is from 40 to 60 nm.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:30:70 (3-methylpyridine: oxygen: $H_2O$) and where the bed temperature was controlled at 320°C . The feed speed of 3-methylpyridine is 0.025 $hr^{-1}$. The product was collected at the outlet of the catalyst bed and analyzed by HPLC and GC. It was found that a conversion of 3-methylpyridine is 88.10%, a selectivity of nicotinic acid is 88.32%, and a selectivity of carbon dioxide is 9.25%.

Example 8 The preparation of the catalyst of the present invention and the process for preparing nicotinic acid by using the catalyst

6.43 g of ammonium meta-vanadate were added into 500 ml water and the solution was heated at 70°C to dissolve ammonium meta-vanadate. Then, 5.46 g of ammonium chromate were added into the solution and stirred for 30 minutes. Into the resultant solution were added 91.41 g titanium oxide (Degussa P-25) and stirred for 1 hour. The mixture was heated to evaporate water and then calcined in an oven at a temperature of 700°C to obtain the catalyst of the present invention, whose composition was shown in Table 1.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:40:175 (3-methylpyridine: oxygen: $H_2O$) and where the bed temperature was controlled at 305°C. The feed speed of 3-methylpyridine is 0.02 $hr^{-1}$. The product was collected at the outlet of the catalyst bed and analyzed by HPLC and GC. It was found that a conversion of 3-methylpyridine is 91.06%, a selectivity of nicotinic acid is 90.91 %, and a selectivity of carbon dioxide is 8.71%.

Example 9 The preparation of the catalyst of the present invention and the process for preparing nicotinic acid by using the catalyst

3.21 g of ammonium meta-vanadate were added into 500 ml water and the solution was heated at 70°C to dissolve ammonium meta-vanadate. Then, 2.73 g of ammonium chromate were added into the solution and stirred for 30 minutes. Into the resultant solution were added 95.71 g titanium oxide (Hembitec K-03) and stirred for 1 hour. The mixture was heated to evaporate water and then calcined in an oven at a temperature of 700°C to obtain the catalyst of the present invention, whose composition was shown in Table 1.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:35:160 (3-methylpyridine: oxygen: $H_2O$) and where the bed temperature was controlled at 265°C. The feed speed of 3-methylpyridine is 0.021 $hr^{-1}$. The product was collected at the outlet of the catalyst bed and analyzed by HPLC and GC. It was found that a conversion of 3-methylpyridine is 92.99%, a selectivity of nicotinic acid is 88.75%, and a selectivity of carbon dioxide is 10.54%.

Example 10 The preparation of the catalyst of the present invention and the process for preparing nicotinic acid by using the catalyst

12.86 g of ammonium meta-vanadate were added into 500 ml water and the solution was heated at 70°C to dissolve ammonium meta-vanadate. Then, 4.99 g of ammonium tungstate were added into the solution and stirred for 30 minutes. Into the resultant solution were added 80.30 g titanium oxide (Hembitec K-03) and stirred for 1 hour. The mixture was heated to evaporate water and then calcined in an oven at a temperature of 600°C to obtain the catalyst of the present invention, which composition was shown in Table 1.

Subsequently, 30g of the prepared catalyst were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:30:160 (3-methylpyridine: oxygen: $H_2O$) and where the bed temperature was controlled at 285°C. The feed speed of 3-methylpyridine is 0.05 $hr^{-1}$. The product was collected at the outlet of the catalyst bed and analyzed by HPLC and GC. It was found that a conversion of 3-methylpyridine is 97.65%, a selectivity of nicotinic acid is 92.58%, and a selectivity of carbon dioxide is 7.23%.

Table 1

| Example No. | Catalyst Composition | Type of titanium oxide | Calcined Temperature (°C) | Reaction Temperature (°C) | Feed Mole Ratio 3-Methylpyridine : $O_2$ : $H_2O$ | 3-Methylpyridine WHSV($h^{-1}$) | Conversion of 3-Methylpyridine (%) | Selectivity of Nicotinic Acid (%) | Selectivity of Carbon Dioxide (%) |
|---|---|---|---|---|---|---|---|---|---|
| 1 | 5% $V_2O_5$/ 5.39%$CrO_3$/$TiO_2$ | K-03 | 700 | 290 | 1 : 45 : 145 | 0.025 | 96.82 | 93.16 | 6.76 |
| 2 | 5% $V_2O_5$/ 2.77%$MoO_3$/$TiO_2$ | K-03 | 600 | 300 | 1 : 40 : 170 | 0.021 | 90.21 | 90.18 | 8.54 |
| 3 | 5% $V_2O_5$/ 2.35%$WO_3$/$TiO_2$ | K-03 | 600 | 285 | 1 : 37 : 160 | 0.028 | 92.83 | 92.22 | 7.11 |
| 4 | 5% $V_2O_5$/ 3.5%$MnO_3$/$TiO_2$ | K-03 | 600 | 305 | 1 : 20 : 150 | 0.025 | 95.68 | 89.88 | 8.98 |
| 5 | 5% $V_2O_5$/ 2.68%$Fe_2O_3$/$TiO_2$ | K-03 | 700 | 310 | 1 : 35 : 330 | 0.02 | 96.78 | 93.13 | 5.56 |
| 6 | 5% $V_2O_5$/ 2.73%$Cr_2O_3$/$TiO_2$ | K-03 | 600 | 300 | 1 : 40 : 170 | 0.021 | 93.54 | 93.16 | 6.39 |
| 7 | 5% $V_2O_5$/ 2.32%$ZnO$/$TiO_2$ | K-03 | 600 | 320 | 1 : 30 : 70 | 0.025 | 88.10 | 88.32 | 9.25 |
| 8 | 5% $V_2O_5$/ 3.59%$CrO_3$/$TiO_2$ | P-24 | 700 | 305 | 1 : 40 : 175 | 0.02 | 91.06 | 90.91 | 8.71 |
| 9 | 2.5% $V_2O_5$/ 1.8%$CrO_3$/$TiO_2$ | K-03 | 700 | 265 | 1 : 35 : 160 | 0.021 | 92.99 | 88.75 | 10.54 |
| 10 | 10% $V_2O_5$/ 4.7%$WO_3$/$TiO_2$ | K-03 | 600 | 285 | 1 : 30 : 160 | 0.05 | 97.65 | 92.58 | 7.23 |

Example 11

30 g of the catalyst prepared in Example 2 were fed into a tube reactor having a diameter of 1 inch and a length of 5 centimeter to obtain a catalyst bed. 3-Methylpyridine was first mixed with air and then with $H_2O$ vapor and then continuously fed into the catalyst bed at a mole ratio of 1:40:170 (3-methylpyridine: oxygen: $H_2O$) and where the bed temperature was controlled at 300°C. The feed speed of 3-methylpyridine is 0.021 hr$^{-1}$. At the outlet of the catalyst bed about 1 liter crude product was collected and analyzed by HPLC and GC. It was found that the contents of 3-methylpyridine and nicotinic acid were 0.0316% and 2.91 %, respectively. The crude product was introduced into a distillation column in which a overhead temperature was set at 97°C and the distillate was condensed in a volume of about 640 ml. The condensate was analyzed by HPLC and found that the content of 3-methylpyridine was 0.047%. From the content of 3-methylpyridine before and after distillation, its recovery rate was calculated and found as 95%. The volume of bottom residue was 360 ml, which was crystallized to get nicotinic acid crystal and the crystal was analyzed. The purity of nicotinic acid by HPLC and found being at least 99%.

From the above Examples, it is known that by using the catalyst of the present invention, which is produced by controlling the crystal size of the active ingredient on the surface of carrier in a certain range through the addition of transition metal oxide, the nicotinic acid could be produced in a high yield and high selectivity. Also, since the crystal size of the active ingredient on the surface of carrier is controlled in a certain range, the catalyst of the present invention exhibits a higher catalytic activity so that desired conversion and selectivity can be achieved by using a lower content of catalyst.

## Claims

1. A process for preparing nicotinic acid, which comprises subjecting a mixture of 3-methylpyridine, oxygen, and water to a vapor phase oxidation at a temperature of from 250°C to 350° C in the presence of a catalyst consisting of, as active ingredients, vanadium oxide ($V_2O_5$) and transition metal oxide both of which are supported on a support, to give the nicotinic acid, wherein the catalyst is an oxide catalyst produced from calcination, wherein the calcinations is carried out at a temperature of from 450 to 800° C, and drying of ammonium meta-vanadate and transition metallate salts supported on a carrier and the crystal size of the active ingredients on the surface of the carrier is controlled in a range of from 40 to 200 nm through use of the transition metal oxide.

2. The process according to claim 1, which further comprises a steps of scrubbing the product stream containing nicotinic acid and un-reacted 3-methylpyridine into water, and distilling the resulting aqueous solution at a overhead temperature of from 96° C to 100°C to distill off and recycle the 3-methylpyridine.

3. The process according to claim 1 or 2, wherein the crystal size of the active ingredients on the surface of the carrier is in a range of from 40 to 100 nm.

4. The process according to claim 1 or 2, wherein a mole ratio of 3-methylpyridine to oxygen is from 1:15 to 1:60, a mole ratio of 3-methylpyridine to water is from 1 :70 to 1:350.

5. The process according to claim 1 or 2, wherein the 3-methylpyridine is fed into the reaction at a WHSV (Weight Hourly Space Velocity) of from 0.01 to 0.1 hr$^{-1}$.

6. The process according to claim 1 or 2, wherein the transition metallate salts are inorganic salts of one or more transition metal selected from the group consisting of chromium, molybdenum, tungsten, manganese, ferric, cobalt, nickel, copper, and zinc.

7. The process according to claim 1 or 2, wherein the transition metallate salts are selected from the group consisting of ammonium chromate, sodium chromate, potassium chromate, calcium chromate, magnesium chromate, chromium nitrate, chromium sulfate, chromium hydroxide, ammonium molybdenate, sodium molybdenate, potassium molybdenate, calcium molybdenate, magnesium molybdenate, ammonium tungstate, sodium tungstate, potassium tungstate, calcium tungstate, magnesium tungstate, ammonium permanganate, sodium permanganate, potassium permanganate, calcium permanganate, magnesium permanganate, ferric nitrate, ferric sulfate, zinc nitrate, and zinc sulfate.

8. The process according to claim 1 or 2, wherein after calcination the amount of vanadium oxide is from 2.5 to 20% by weight and the amount of transition metal oxide is from 0.1 to 10% by weight, based on the total weight of the vanadium oxide, transition metal oxide, and the carrier.

9. The process according to claim 1 or 2, wherein the carrier is titanium oxide and/or aluminum oxide.

10. The process according to claim 9, wherein the carrier is titanium oxide.

11. An oxidation catalyst, which is consisting of vanadium oxide ($V_2O_5$) and transition metal oxide both of which are supported on a carrier, in which a crystal size of the active ingredients on the surface of the carrier is in a range of from 40 to 200 nm.

12. The oxidation catalyst according to claim 11, wherein the crystal size of the active ingredients on the surface of the carrier is in a range of from 40 to 100 nm.

13. The oxidation catalyst according to claim 11, wherein the transition metal oxide is one or more metal oxides selected from the group consisting of chromium oxide, molybdenum oxide, tungsten oxide, manganese oxide, ferric oxide, cobalt oxide, nickel oxide, cupric oxide, and zinc oxide.

14. The oxidation catalyst according to claim 11, wherein carrier is titanium oxide and/or aluminum oxide.

15. The oxidation catalyst according to claim 14, wherein the carrier is titanium oxide.

16. The oxidation catalyst according to claim 11, wherein the amount of vanadium oxide is from 2.5 to 20% by weight and the amount of transition metal oxide is from 0.1 to 10% by weight, based on the total weight of the vanadium oxide, transition metal oxide, and the carrier.

**Patentansprüche**

1. Verfahren zum Herstellen von Nikotinsäure, das das Unterwerfen einer Mischung von 3-Methylpyridin, Sauerstoff und Wasser einer Dampfphasenoxidation bei einer Temperatur von 250°C bis 350°C in Gegenwart eines Katalysators, bestehend aus Vanadiumoxid ($V_2O_5$) und Übergangsmetalloxid als aktive Bestandteile, wobei beide auf einem Träger aufgebracht sind, umfasst, um Nikotinsäure zu ergeben, wobei der Katalysator ein Oxidkatalysator ist, der durch Kalzinierung hergestellt wurde, wobei die Kalzinierung bei einer Temperatur von 450 bis 800°C durchgeführt wird, und Trocknen von Ammoniummetavanadat und Übergangsmetallsalzen, die auf einem Träger aufgebracht sind, und die Kristallgröße der aktiven Bestandteile auf der Oberfläche des Trägers in einem Bereich von 40 bis 200 nm durch Verwendung des Übergangsmetalloxides gesteuert wird.

2. Verfahren gemäß Anspruch 1, das des Weiteren einen Schritt des Waschens des Produktstromes, der die Nikotinsäure und unreagiertes 3-Methylpyridin enthält, in Wasser und Destillieren der resultierenden wässrigen Lösung bei einer Kopftemperatur von 96 bis 100°C, um das 3-Methylpyridin abzudistillieren und zu recyceln, umfasst.

3. Verfahren gemäß Anspruch 1 oder 2, wobei die Kristallgröße der aktiven Bestandteile auf der Oberfläche des Trägers in einem Bereich von 40 bis 100 nm liegt.

4. Verfahren gemäß Anspruch 1 oder 2, wobei ein Mol-Verhältnis von 3-Methylpyridin zu Sauerstoff von 1:15 bis 1:60, ein Mol-Verhältnis von 3-Methlypyridin zu Wasser von 1:70 bis 1:350 beträgt.

5. Verfahren gemäß Anspruch 1 oder 2, wobei das 3-Methylpyridin in die Reaktion bei einer WHSV (stündliche Gewichtskatalysatorbelastung) von 0,01 bis 0,1 hr-[1] zugeführt wird.

6. Verfahren gemäß Anspruch 1 oder 2, wobei die Übergangsmetallsalze anorganische Salze eines oder mehrerer Übergangsmetalle sind, ausgewählt aus der Gruppe, bestehend aus Chrom, Molybdän, Wolfram, Mangan, Eisen (III), Kobalt, Nickel, Kupfer und Zink.

7. Verfahren gemäß Anspruch 1 oder 2, wobei die Übergangsmetallsalze ausgewählt sind aus der Gruppe, bestehend aus Ammoniumchromat, Natriumchromat, Kaliumchromat, Kalziumchromat, Magnesiumchromat, Chromnitrat, Chromsulfat, Chromhydroxid, Ammoniummolybdat, Natriummolybdat, Kaliummolybdat, Kalziummolybdat, Magnesiummolybdat, Ammoniumwolframat, Natriumwolframat, Kaliumwolframat, Kalziumwolframat, Magnesiumwolframat, Ammoniumpermanganat, Natriumpermanganat, Kaliumpermanganat, Kalziumpermanganat, Magnesiumpermanganat, Eisen(III)-nitrat, Eisen(III)-sulfat, Zinknitrat und Zinksulfat.

8. Verfahren gemäß Anspruch 1 oder 2, wobei nach der Kalzinierung die Menge an Vanadiumoxid von 2,5 bis 20 Gew.-% und die Menge an Übergangsmetalloxid von 0,1 bis 10 Gew.-%, basierend auf dem Gesamtgewicht des Vanadiumoxids, Übergangsmetalloxids und des Trägers, beträgt.

9. Verfahren gemäß Anspruch 1 oder 2, wobei der Träger Titanoxid und/oder Aluminiumoxid ist.

10. Verfahren gemäß Anspruch 9, wobei der Träger Titanoxid ist.

11. Oxidationskatalysator, der aus Vanadiumoxid ($V_2O_5$) und Übergangsmetalloxid besteht, wobei beide auf einem Träger aufgebracht sind, in welchem eine Kristallgröße des aktiven Bestandteils auf der Oberfläche des Trägers in einem Bereich von 40 bis 200 nm ist.

12. Oxidationskatalysator gemäß Anspruch 11, wobei die Kristallgröße der aktiven Bestandteile auf der Oberfläche des Trägers in einem Bereich von 40 bis 100 nm ist.

13. Oxidationskatalysator gemäß Anspruch 11, wobei das Übergangsmetalloxid eines oder mehrere Metalloxide, ausgewählt aus der Gruppe, bestehend aus Chromoxid, Molybdänoxid, Wolframoxid, Manganoxid, Eisen(III)-oxid, Kobaltoxid, Nickeloxid, Kupfer(II)-oxid und Zinkoxid, ist.

14. Oxidationskatalysator gemäß Anspruch 11, wobei der Träger Titanoxid und/oder Aluminiumoxid ist.

15. Oxidationskatalysator gemäß Anspruch 14, wobei der Träger Titanoxid ist.

16. Oxidationskatalysator gemäß Anspruch 11, wobei die Menge an Vanadiumoxid von 2,5 bis 20 Gew.-% und die Menge an Übergangsmetalloxid von 0,1 bis 10 Gew.-%, basierend auf dem Gesamtgewicht des Vanadiumoxids, des Übergangsmetalloxids und des Trägers, beträgt.


**Revendications**

1. Processus pour préparer de l'acide nicotinique, qui comprend la soumission d'un mélange de 3-méthylpyridine, d'oxygène et d'eau à une oxydation en phase vapeur à une température comprise de 250° C à 350° C en présence d'un catalyseur constitué de, en tant que composés actifs, de l'oxyde de vanadium ($V_2O_5$) et un oxyde de métal de transition les deux étant supportés sur un support, pour donner l'acide nicotinique, dans lequel le catalyseur est un catalyseur de type oxide produit à partir d'une calcination, dans lequel la calcination est effectuée à une température de 450 à 800° C, et le séchage de métavanadate d'ammonium et de sels de métallate de transition supportés sur un support et la taille des cristaux des composés actifs sur la surface du support est contrôlée dans une plage de 40 à 200 nm au moyen de l'utilisation de l'oxyde de métal de transition.

2. Processus selon la revendication 1, qui comprend une étape de lavage dans l'eau du flux de produit contenant de l'acide nicotinique et de la 3-méthylpyridine qui n'a pas réagi et à distiller la solution aqueuse résultante à une température de tête de 96° C à 100° C pour distiller et recycler la 3-méthylpyridine.

3. Processus selon la revendication 1 ou 2, dans lequel la taille des cristaux des composés actifs sur la surface du support est dans une plage de 40 à 100 nm.

4. Processus selon la revendication 1 ou 2, dans lequel un rapport molaire entre la 3-méthylpyridine et l'oxygène est de 1:15 à 1:60, un rapport molaire entre la 3-méthylpyridine et l'eau est de 1:70 à 1:350.

5. Processus selon la revendication 1 ou 2, dans lequel la 3-méthylpyridine est alimentée dans la réaction à une WHSV (Vitesse Spatiale Horaire en Poids) de 0,01 à 0 , 1 hr[-1].

6. Processus selon la revendication 1 ou 2, dans lequel les sels de métallate de transition sont des sels inorganiques d'un ou de plusieurs métaux de transition sélectionnés dans le groupe constitué par le chrome, le molybdène, le tungstène, le manganèse, le fer ferrique, le cobalt, le nickel, le cuivre et le zinc.

**7.** Processus selon la revendication 1 ou 2, dans lequel les sels de métallate de transition sont sélectionnés dans le groupe constitué par le chromate d'ammonium, le chromate de sodium, le chromate de potassium, le chromate de calcium, le chromate de magnésium, le nitrate de chrome, le sulfate de chrome, l'hydroxyde de chrome, le molybdate d'ammonium, le molybdate de sodium, le molybdate de potassium, le molybdate de calcium, le molybdate de magnésium, le tungstate d'ammonium, le tungstate de sodium, le tungstate de potassium, le tungstate de calcium, le tungstate de magnésium, le permanganate d'ammonium, le permanganate de sodium, le permanganate de potassium, le permanganate de calcium, le permanganate de magnésium, le nitrate ferrique, le sulfate ferrique, le nitrate de zinc et le sulfate de zinc.

**8.** Processus selon la revendication 1 ou 2, dans lequel après calcination la quantité d'oxyde de vanadium est de 2,5 à 20 % en poids et la quantité d'oxyde de métal de transition est de 0,1 et 10 % en poids, basées sur le poids total de l'oxyde de vanadium, de l'oxyde de métal de transition et du support.

**9.** Processus selon la revendication 1 ou 2, dans lequel le support est de l'oxyde de titane et/ou de l'oxyde d'aluminium.

**10.** Processus selon la revendication 9, dans lequel le support est de l'oxyde de titane.

**11.** Catalyseur d'oxydation, qui est constitué d'oxyde de vanadium ($V_2O_5$) et d' un oxyde de métal de transition les deux étant supportés sur un support, dans lequel une taille des cristaux des composés actifs sur la surface du support est dans une plage de 40 à 200 nm.

**12.** Catalyseur d'oxydation selon la revendication 11, dans lequel la taille des cristaux des composés actifs sur la surface du support est dans une plage de 40 à 100 nm.

**13.** Catalyseur d'oxydation selon la revendication 11, dans lequel l'oxyde de métal de transition est un ou plusieurs oxydes de métaux sélectionnés dans le groupe constitué par l'oxyde de chrome, l'oxyde de molybdène, l'oxyde de tungstène, l'oxyde de manganèse, l'oxyde ferrique, l'oxyde de cobalt, l'oxyde de nickel, l'oxyde de cuivrique et l'oxyde de zinc.

**14.** Catalyseur d'oxydation selon la revendication 11, dans lequel le support est de l'oxyde de titane et/ou de l'oxyde d'aluminium.

**15.** Catalyseur d'oxydation selon la revendication 14, dans lequel le support est de l'oxyde de titane.

**16.** Catalyseur d'oxydation selon la revendication 11, dans lequel la quantité d'oxyde de vanadium est comprise entre 2,5 et 20 % en poids et la quantité d'oxyde de métal de transition est comprise entre 0,1 et 10 % en poids, basées sur le poids total de l'oxyde de vanadium, de l'oxyde de métal de transition, et du support.

Figure 1 （×25,000）

Figure 2 （×25,000）

Figure 3 (×100,000)

Figure 4 (×25,000)

Figure 5 (×10,000)

Figure 6 (×25,000)

Figure 7 （×25,000）

Figure 8 （×25,000）

Figure 9 (×25,000)

Figure 10 (×25,000)

CCP  SEI  5.0kV  X25,000  1μm  WD 7.2mm

**Figure 11 (×25,000)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 568889 A
- US 4217457 A
- US 5700944 A
- US 3803156 A
- US 5719045 A
- US 5728837 A
- US 6229018 B


**Non-patent literature cited in the description**

- *JOURNAL OF CATALYSIS,* vol. 192 (1), 1-10